# EUROPÄISCHE PATENTSCHRIFT

(11) **EP 2 226 028 B1**
(45) Veröffentlichungstag und Bekanntmachung des Hinweises auf die Patenterteilung: **04.12.2013**
(21) Anmeldenummer: 10154764.4
(22) Anmeldetag: 26.02.2010
(51) Int. Cl.: A61B 17/88, A61B 17/82

(54) **Chirurgisches Schneidinstrument**
Surgical cutting instrument
Instrument de coupe chirurgical

(30) Priorität: 06.03.2009 DE 102009013368
(43) Veröffentlichungstag der Anmeldung: 08.09.2010
(73) Patentinhaber: Aesculap AG, 78532 Tuttlingen (DE)
(72) Erfinder: Morales, Pedro, 78532, Tuttlingen-Nendingen (DE); Weißhaupt, Dieter, 78194, Immendingen (DE)
(74) Vertreter: Hoeger, Stellrecht & Partner Patentanwälte

(56) Entgegenhaltungen:
- EP-A1- 1 138 266
- EP-A2- 1 201 194
- WO-A2-02/089659
- US-A1- 2008 249 532

## Beschreibung

Die Erfindung betrifft ein chirurgisches Schneidinstrument zum Durchtrennen eines insbesondere stiftförmigen Schneidguts, welches Schneidinstrument zwei relativ zueinander bewegbare, jeweils eine Schneide aufweisende Schneidbacken umfasst, welche Schneiden in einer Anlegestellung voneinander beabstandet sind und in einer Schneidstellung längs einer Schneidlinie aneinander anliegen oder im Wesentlichen aneinander anliegen zum Durchtrennen des Schneidguts, wobei das Schneidinstrument eine Halteeinrichtung zum Halten eines abgetrennten Teils des Schneidguts nach dem Durchtrennen desselben umfasst, wobei die Halteeinrichtung eine Schneidgutaufnahme für einen abzutrennenden Teil des Schneidguts aufweist und wobei die Halteeinrichtung mindestens ein Halteglied zum Halten des abzutrennenden Teils in der Schneidgutaufnahme vor und/oder nach dem Durchtrennen des Schneidguts umfasst, wobei eine Verformungseinrichtung zum Verformen des in der Schneidgutaufnahme gehaltenen und vom Schneidgut abzutrennenden Teils desselben vorgesehen ist und wobei die Verformungseinrichtung mindestens ein Verformungselement umfasst, welches relativ zur Schneidgutaufnahme beim Übergang von der Anlegestellung in die Schneidstellung bewegbar ist und wobei das mindestens eine Verformungselement mindestens teilweise in die in der Anlegestellung definierte Schneidgutaufnahme hineinbewegbar ist beim Übergang von der Anlegestellung in die Schneidstellung.

Chirurgische Schneidinstrumente der eingangs beschriebenen Art werden insbesondere dazu verwendet, überstehende Abschnitte von zwei Anlageelemente verbindenden Verbindungsstiften einer Verbindungsvorrichtung zum Schließen eines durchtrennten Brustbeins, welches insbesondere für eine Operation am offenen Herzen durchtrennt wird, abzutrennen. Derartige Verbindungsvorrichtungen sind beispielsweise in der DE 103 26 690 B4 beschrieben. Bekannte Schneidinstrumente, die beispielsweise in Form von Schneid- oder Kneifzangen ausgebildet sind, erfordern es, dass ein Operateur, der einen überschüssigen Teil des die beiden Anlageelemente zum Verbinden des Sternums aneinander führenden und miteinander verbindenden Verbindungselements oder -stifts abtrennen möchte, den abzutrennenden Teil hält. Hält der Operateur den abzutrennenden Teil des Schneidguts nicht, wird er üblicherweise durch den beim Schneiden auf das Schneidgut wirkenden Schneidimpuls unkontrolliert weggesprengt. Um Verletzungen am Patienten, am Anwender oder Dritten, bei einer Operation anwesender Personen zu vermeiden, bleibt dem Operateur nichts anderes übrig, als den überstehenden Teil des Verbindungselements mit der Hand zu halten oder von einer ihm assistierenden Person von Hand oder mit einem Halteinstrument halten zu lassen.

Aus der US 2008/0249532 A1, der EP 1 138 266 A1 sowie der WO 02/089659 A1 sind chirurgische Schneidinstrumente zum Durchtrennen eines insbesondere stiftförmigen Schneidguts bekannt.

Es ist daher eine Aufgabe der vorliegenden Erfindung, ein chirurgisches Schneidinstrument der eingangs beschriebenen Art so zu verbessern, dass das Durchtrennen eines Schneidguts für einen Operateur einfacher und sicherer wird.

Diese Aufgabe wird bei einem chirurgischen Schneidinstrument der eingangs beschriebenen Art erfindungsgemäß dadurch gelöst, dass mindestens ein Teil des mindestens einen Verformungselement um eine Verformungselementlängsachse rotierbar gelagert ist.

Die erfindungsgemäß vorgeschlagene Weiterbildung bekannter chirurgischer Schneidinstrumente macht das Halten des Schneidguts, beispielsweise eines Verbindungselements einer Sternumverbindungsvorrichtung, von Hand oder mittels einer Hilfseinrichtung durch einen Operateur oder eine ihm assistierende Person überflüssig. Das Schneidinstrument kann insbesondere direkt an das Schneidgut angesetzt werden, wobei das Schneidinstrument den abgetrennten Teil des Schneidguts nach dem Durchtrennen des Schneidguts automatisch hält. So kann vermieden werden, dass es in Folge des beim Durchtrennen wirkenden Schneidimpulses unkontrolliert weggesprengt wird. Insbesondere bei einem Schneidgut aus Metall, zum Beispiel bei stiftförmigen Verbindungselementen von Stemumverbindungsvorrichtungen, kann so die Gefahr ausgeschlossen werden, den Patienten oder andere im Operationssaal anwesende Personen zu verletzen. Die Halteinrichtung kann insbesondere auch zum Halten des Schneidguts insgesamt am Schneidinstrument dienen, also zum Halten noch vor dem eigentlichen Schneid- oder Trennvorgang. Dadurch, dass die Halteeinrichtung eine Schneidgutaufnahme für einen abzutrennenden Teil des Schneidguts aufweist, ist es so insbesondere möglich, das Schneidinstrument in definierter Weise am Schneidgut anzulegen. Der abzutrennende Teil des Schneidguts kann insbesondere in der Schneidgutaufnahme aufgenommen und mit der Halteeinrichtung beispielsweise darin gehalten werden. Insbesondere kann die Halteeinrichtung ausgebildet sein in Form einer Klemmeinrichtung zum klemmenden Halten eines abzutrennenden und/oder abgetrennten Teils des Schneidguts. Besonders einfach wird der Aufbau des Schneidinstruments dadurch, dass die Halteeinrichtung mindestens ein Halteglied zum Halten des abzutrennenden Teils in der Schneidgutaufnahme vor und/oder nach dem Durchtrennen des Schneidguts umfasst. Das mindestens eine Halteglied ermöglicht es, den abzutrennenden Teil des Schneidguts in definierter Weise zu halten, und zwar in der Schneidgutaufnahme. Das abzutrennende Schneidgut kann insbesondere vor dem Durchtrennen, nach dem Durchtrennen sowie vor und nach dem Durchtrennen in der Schneidgutaufnahme gehalten werden. Vorteilhafterweise umfasst das Schneidinstrument eine Verformungseinrichtung zum Verformen des in der Schneidgutaufnahme gehaltenen und vom Schneidgut abzutrennenden Teils desselben. Insbesondere kann ein Verformen des vom Schneidgut abzutrennenden Teils desselben genutzt werden, um es sicher in der Schneidgutaufnahme zu halten. Beispielsweise kann es in der Schneidgutaufnahme klemmend gehalten sein mittels der Halteeinrichtung und, während die Halteeinrichtung das Schneidgut hält, zusätzlich verformt werden, um dadurch insbesondere auch das Schneidgut noch besser und sicherer zu halten. Auf einfache Weise lässt sich das Schneidgut dadurch verformen, dass die Verformungseinrichtung mindestens ein Verformungselement umfasst, welches relativ zur Schneidgutaufnahme beim Übergang von der Anlegestellung in die Schneidstellung bewegbar ist. So kann es direkt oder indirekt auf das in der Schneidgutaufnahme gehaltene Schneidgut einwirken, um es zu verformen. Günstigerweise ist das mindestens eine Verformungselement mindestens teilweise in die in der Anlegestellung definierte Schneidgutaufnahme hineinbewegbar beim Übergang von der Anlegestellung in die Schneidstellung. Auf diese Weise kann das mindestens eine Verformungselement direkt auf das in der Schneidgutaufnahme in der Anlegestellung gehaltene Schneidgut einwirken, um es zu verformen. Selbstverständlich ist es möglich, dass sich aufgrund der Bewegung des Verformungselements die Schneidgutaufnahme ändert, insbesondere kann sie schmaler werden. Gemäß der Erfindung ist mindestens ein Teil des mindestens einen Verformungselements um eine Verformungselementlängsachse rotierbar gelagert ist. Es ist also nicht zwingend erforderlich das Verformungselement als Ganzes rotierbar zu lagern. Dies ermöglicht es, den nicht rotierbar gelagerten Teil insbesondere unbeweglich am Schneidinstrument festzulegen. Vorzugsweise ist der mindestens eine Teil des Verformungselements, welcher rotierbar gelagert ist, derart angeordnet, dass er die Schneidgutaufnahmeebene berührt.

Vorzugsweise ist die Verformungseinrichtung derart ausgebildet, dass der abzutrennende Teil des Schneidguts beim Übergang von der Anlegestellung in die Schneidstellung verformbar ist. Durch eine Verformung kann das Schneidgut noch besser und sicherer mittels der Halteeinrichtung gehalten werden. Insbesondere kann ein Verklemmen des Schneidguts in oder an der Halteeinrichtung durch ein Verformen desselben noch erhöht werden.

Um das mindestens eine Verformungselement nur wenig bewegen zu müssen, damit es das Schneidgut in der Schneidgutaufnahme verformen kann, ist es vorteilhaft, wenn das mindestens eine Verformungselement die Schneidgutaufnahme mindestens teilweise begrenzt.

Besonders einfach wird der Aufbau des Schneidinstruments, wenn die Schneidgutaufnahme spiegelsymmetrisch zu einer Spiegelebene ausgebildet ist und wenn die Spiegelebene eine von den Schneidbacken definierte Längsachse enthält.

Um direkt und sicher auf das in der Schneidgutaufnahme gehaltene Schneidgut einwirken zu können, ist es günstig, wenn das mindestens eine Verformungselement die Schneidgutaufnahme entlang einer die Schneidgutaufnahme begrenzenden Schneidgutaufnahmeebene berührt, die parallel zur Spiegelebene verläuft. Insbesondere kann die Berührung der Schneidgutaufnahmeebene durch das Verformungselement punkt- oder linienförmig oder im Wesentlichen linienförmig sein. Insbesondere kann eine Berührlinie des Verformungselements mit der Schneidgutaufnahmeebene quer oder senkrecht zu einer Längsachse des Schneidinstruments verlaufen.

Einfach und sicher Verformen lässt sich das Schneidgut in der Schneidgutaufnahme, wenn ein Abstand der Schneidgutaufnahmeebene von der Spiegelebene in der Anlegestellung größer ist als in der Schneidstellung. Mit anderen Worten bedeutet dies, dass eine Begrenzung der Schneidgutaufnahmeebene, definiert durch einen Kontakt zwischen dem mindestens einem Verformungselement und der Schneidgutaufnahme, beim Übergang von der Anlegestellung in die Schneidstellung wandert, und zwar in Richtung auf die Spiegel- oder Symmetrieebene des Instruments beziehungsweise der Schneidbacken hin. Dadurch lässt sich das Schneidgut in der Schneidgutaufnahme durch erhöhte Klemmkräfte noch sicherer halten und/oder durch entsprechende Kraftbeaufschlagung verformen.

Um das Schneidgut noch einfacher und sicherer verformen zu können, ist es vorteilhaft, wenn die Verformungseinrichtung zwei Verformungselemente umfasst.

Günstig ist es, wenn die zwei Verformungselemente bezogen zur Spiegelebene unsymmetrisch angeordnet sind. Insbesondere können sie in einem unterschiedlichen Abstand zu einem distalen Ende des Instruments angeordnet sein. Dadurch kann mittels der Verformungseinrichtung an unterschiedlichen Stellen gegen das in der Schneidgutaufnahme befindliche Schneidgut gerückt werden, um es zum Beispiel zu verformen oder noch sicherer zu halten. Vorteilhafterweise sind die zwei Verformungselemente auf unterschiedlichen Seiten der Spiegelebene positioniert. Dies ermöglicht es, in zueinander entgegengesetzten Richtungen gegen das in der Schneidgutaufnahme enthaltene Schneidgut zu drücken, um es entweder optimiert zu halten und/oder zu verformen.

Vorzugsweise ist ein erstes Verformungselement von einem distalen Ende des Schneidinstruments weiter beabstandet als ein zweites Verformungselement. Auf diese Weise ist es möglich, insbesondere eine Dreipunktauflage zu schaffen, nämlich das Schneidgut beim Durchtrennen mit den Schneiden sowie den beiden Verformungselementen zu halten. Insbesondere kann es dabei vorteilhaft sein, wenn die zwei Verformungselemente auf unterschiedlichen Seiten der Spiegelebene positioniert sind, so dass drei die Dreipunktauflage definierende Berührpunkte nicht auf einer Geraden liegen.

Der Aufbau des Schneidinstruments wird besonders einfach, wenn das mindestens eine Verformungselement in Form eines Haltebolzens ausgebildet ist. Insbesondere kann der Haltebolzen zylindrisch geformt sein.

Damit mit dem Haltebolzen gezielt gegen das Schneidgut gedrückt werden kann, ist es vorteilhaft, wenn der Haltebolzen eine Haltebolzenlängsachse definiert, welche parallel oder im Wesentlichen parallel zur Schneidlinie verläuft. Insbesondere kann die Haltebolzenlängsachse senkrecht zu einer vom Schneidinstrument definierten Längsachse verlaufen.

Besonders einfach wird der Aufbau des mindestens einen Verformungselements, wenn es als Ganzes rotierbar um die Verformungselementlängsachse gelagert ist.

Einerseits eine gewünschte Stabilität und andererseits eine geforderte Beweglichkeit lässt sich bei der Verformungseinrichtung insbesondere dadurch erreichen, dass das mindestens eine Verformungselement ein Lagerglied und ein Verformungsglied umfasst und dass das Verformungsglied am Lagerglied um die Verformungselementlängsachse rotierbar gelagert ist.

Ein konstruktiv einfacher Aufbau des mindestens einen Verformungselements kann insbesondere dadurch erreicht werden, dass das Lagerglied in Form eines zylindrischen Lagerbolzens ausgebildet ist und dass das Verformungsglied in Form einer auf dem Lagerbolzen rotierbar gelagerten Verformungshülse ausgebildet ist. So kann sich das Schneidgut einfach und sicher entlang des Verformungsglieds bewegen, wenn es aufgrund einer Materialverformung beim Durchtrennen etwas in proximaler Richtung bewegt wird. Insbesondere lassen sich so Bewegungskräfte im Wesentlichen spiel- und reibungsfrei aufnehmen bei entsprechender Lagerung der Verformungshülse am Lagerbolzen.

Der Aufbau des Schneidinstruments kann weiter vereinfacht werden, wenn die Schneidbacken relativ zueinander um eine erste Schwenkachse verschwenkbar angeordnet oder gelagert sind. Insbesondere können sie direkt um die erste Schwenkachse aneinander bewegbar gelagert sein. Zum Überführen des Schneidinstruments von der Anlegestellung in die Schneidstellung müssen die Schneidbacken dann nur um die erste Schenkachse verschwenkt werden.

Ein besonders kompakter Aufbau des Schneidinstruments kann beispielsweise dadurch erreicht werden, dass das mindestens eine Verformungsglied um die erste Schwenkachse verschwenkbar gelagert ist. Insbesondere ist es so denkbar, das mindestens eine Verformungsglied an einem der relativ zueinander um die erste Schwenkachse verschwenkbaren Schneidbacken anzuordnen oder zu lagern.

Um die Schneidbacken in definierter Weise relativ zueinander verschwenken zu können, ist es vorteilhaft, wenn das Schneidinstrument zwei um die erste Schwenkachse verschwenkbar aneinander gelagerte Schwenkhebel umfasst, an denen jeweils eine Schneidbacke angeordnet oder ausgebildet ist. Insbesondere lassen sich so auf einfache Weise Über- beziehungsweise Untersetzungen für eine Betätigungskraft definieren in Abhängigkeit von Hebellängen und Orientierungen derselben mit Bezug zur ersten Schwenkachse.

Um die Zahl der insgesamt beweglichen Teile am Schneidinstrument besonders klein zu halten, ist es günstig, wenn das mindestens eine Verformungselement an den Schwenkhebeln angeordnet oder ausgebildet ist.

Auf einfache Weise kann ein Abstand zwischen zwei von zwei Verformungselementen definierten Schneidgutaufnahmeebenen verringert werden, wenn alle Verformungselemente an nur einem der beiden Schwenkhebel angeordnet oder ausgebildet sind.

Gemäß einer weiteren bevorzugten Ausführungsform der Erfindung kann vorgesehen sein, dass das Schneidinstrument eine Schneidimpulsaufnahmeeinrichtung zum Aufnehmen eines beim Durchtrennen zumindest auf einen abgetrennten Teil des Schneidguts wirkenden Schneidimpulses umfasst. Eine derartige Ausgestaltung kann insbesondere auch bei einem Schneidinstrument der eingangs beschriebenen Art vorgesehen sein. Die Schneidimpulsaufnahmeeinrichtung ermöglicht es, den beim Durchtrennen wirkenden Schneidimpuls aufzunehmen, so dass ein unkontrolliertes Wegsprengen des abgetrennten Teils des Schnittguts vermieden werden kann.

Zum Aufnehmen des Schneidimpulses ist es günstig, wenn die Schneidimpulsaufnahmeeinrichtung mindestens ein Impulsaufnahmeelement aufweist, welches bewegbar am Schneidinstrument gelagert ist und die Schneidgutaufnahme mindestens teilweise begrenzt. So kann insbesondere ein Schneidimpuls vom Impulsaufnahmeelement aufgenommen werden, welches in Folge einer Bewegung des Schneidelements beispielsweise direkt bewegt werden und so den Schneidimpuls aufnehmen kann.

Günstig ist es, wenn das mindestens eine Impulsaufnahmeelement in Folge einer Bewegung des vom Schneidgut abzutrennenden Teils in proximaler Richtung bewegbar oder rotierbar ist. So kann sichergestellt werde, dass der Schneidimpuls nicht in distaler Richtung wirken kann, also insbesondere nicht in Richtung auf einen Patienten hin, was zu unerwünschten Belastungen desselben führen könnte. Vielmehr kann der in proximaler Richtung wirkende Schneidimpuls durch das Schneidinstrument aufgenommen werden, so dass der abzutrennende Teil des Schneidguts auch nicht in Richtung auf eine Bedienperson, also zum Beispiel einen Operateur, wirken und das abgetrennte Schneidgut in diese Richtung wegschleudern oder wegsprengen kann.

Besonders einfach und kompakt lässt sich das Schneidinstrument ausbilden, wenn das mindestens eine Impulsaufnahmeelement durch das mindestens eine Verformungselement gebildet wird. Dieses kann dann gleichzeitig zwei Funktionen erfüllen, nämlich zum einen das in der Schneidgutaufnahme enthaltene Schneidgut zu verformen und zum anderen einen beim Durchtrennen des Schneidguts wirkenden Schneidimpuls aufzunehmen.

Günstigerweise wird das mindestens eine Impulsaufnahmeelement durch das mindestens eine Verformungsglied gebildet. So ist es insbesondere möglich, das Verformungsglied stabil am Schneidinstrument mittels des Lagerglieds zu halten, welches beispielsweise eine Verformungskraft mittels des Verformungsglieds auf das Schneidgut übertragen kann, wobei der Schneidimpuls aufgrund der beweglichen Lagerung des Verformungsglieds am Lagerelement aufgenommen werden kann.

Eine kontinuierliche Schneidimpulsaufnahme, insbesondere unabhängig von einer Verformungs- oder Verschiebungslänge des Schneidguts in der Schneidgutaufnahme, kann auf einfach Weise dadurch erreicht werden, dass das mindestens eine Impulsaufnahmeelement um eine Rotationsachse rotierbar am Schneidinstrument gelagert ist. Abhängig von einem Verformungs- oder Bewegungsweg des Schneidguts in der Schneidgutaufnahme kann so das Impulsaufnahmeelement um einen kleineren oder größeren Drehwinkel um die Rotationsachse rotieren. Eine Bewegungsbegrenzung, das heißt eine Begrenzung eines Bewegungsweges, wie es beispielsweise bei einem verschiebbar gelagerten Impulsaufnahmeelement der Fall wäre, kann so vermieden werden. Das Schneidinstrument eignet sich somit universell für ein beliebiges Schneidgut, und zwar unabhängig von dessen Länge und unabhängig von einem Bewegungsweg.

Günstigerweise verläuft die Rotationsachse parallel zur ersten Schwenkachse. So kann insbesondere auch eine Abrollbewegung des Schneidguts am Impulsaufnahmeelement erfolgen, wenn das mindestens eine Verformungselement um die erste Schwenkachse verschwenkt wird, beispielsweise zum Verformen des Schneidguts.

Ein besonders einfacher und kompakter Aufbau des Schneidinstruments kann insbesondere dadurch erreicht werden, dass die Rotationsachse durch die Haltebolzenlängsachse definiert wird.

Um besonders gute Schneidergebnisse zu erzielen, ist es vorteilhaft, wenn jede Schneide eine Schneidkante aufweist. Alternativ wäre es denkbar, statt einer der beiden Schneiden ein ambossartig geformtes Gegenlager auszubilden und nur an der verbleibenden Schneide eine Schneidkante vorzusehen.

Um eine optimale Durchtrennung des Schneidguts zu ermöglichen, ist es günstig, wenn die Schneidkanten in einer Schneidstellung des Schneidinstruments linienförmig oder im Wesentlichen linienförmig aneinander anliegen und die Schneidlinie definieren.

Vorzugsweise weist jede Schneide eine erste, in distaler oder im Wesentlichen in distaler Richtung weisende Seitenfläche und eine zweite, in proximaler oder im Wesentlichen in proximaler Richtung weisende Seitenfläche auf. Diese Ausgestaltung ermöglicht es, mit den Schneiden quer oder im Wesentlichen quer zu einer Längsrichtung des Schneidinstruments das Schneidgut, das sich in der Schneidgutaufnahme befindet, zu durchtrennen.

Günstigerweise definiert die erste Seitenfläche eine erste Schneidebene, welche in der Schneidstellung eine vom Schneidinstrument definierte Längsachse senkrecht oder im Wesentlichen senkrecht schneidet. Auf diese Weise kann ein in distaler Richtung gerichteter Schneidimpuls minimiert oder sogar ganz ausgeschlossen werden.

Vorzugsweise definiert die zweite Seitenfläche eine zweite Schneidebene, welche in der Schneidstellung relativ zu einer vom Schneidinstrument definierten Längsachse geneigt ist. So kann gezielt ein Schneidimpuls in proximaler Richtung abgeleitet werden, wo er optional mit der Schneidimpulsaufnahmeeinrichtung aufgenommen oder abgefangen werden kann.

Um das Schneidgut in definierter Weise durchtrennen zu können, ist es vorteilhaft, wenn die beiden Seitenflächen der beiden Schneiden in der Schneidstellung eine in proximaler Richtung weisende keilförmige Vertiefung begrenzen und wenn die Vertiefung einen Öffnungswinkel definiert. Durch die Ausbildung einer keilförmigen Vertiefung in der Schneidstellung kann ein Schneidimpuls in definierter Weise in proximaler Richtung abgeleitet werden.

Günstigerweise weist der Öffnungswinkel einen Wert im Bereich von 30° bis 160° auf. Ein solcher Öffnungswinkel, aufgrund entsprechend geformter zweiter Seitenflächen der Schneiden, ermöglicht eine sichere Durchtrennung des Schneidguts.

Für eine optimale Ableitung des Schneidimpulses in proximaler Richtung ist es vorteilhaft, wenn der Öffnungswinkel einen Wert im Bereich von 60° bis 120° aufweist. Vorzugsweise beträgt der Öffnungswinkel 90°.

Gemäß einer weiteren bevorzugten Ausführungsform der Erfindung kann vorgesehen sein, dass ein distales Ende des Schneidinstruments in der Schneidstellung eine nutförmige Ausnehmung aufweist und dass die ersten oder zweiten Schneidflächen die Ausnehmung mindestens teilweise definieren oder begrenzen. Eine solche Ausgestaltung hat den Vorteil, dass dann, wenn ein distales Ende des Schneidinstruments beispielsweise direkt an ein Anlageelement einer Verbindungseinrichtung zum Verbinden des Sternums angelegt wird, nach dem Durchtrennen des Verbindungselements mit dem Schneidinstrument noch ein definierter Überstand bleibt. Mit anderen Worten wird das Schneidgut in definiertem Abstand zu einem distalen Ende des Schneidinstruments durchtrennt.

Günstigerweise ist die Schneidkante von einem distalen Ende des Schneidinstruments beabstandet. So kann auf einfache und reproduzierbare Weise das Schneidgut in immer gleichem Abstand zu einem distalen Ende des Schneidinstruments durchtrennt werden.

Um das Schneidinstrument einfach und sicher handhaben zu können, ist es günstig, wenn es einen ein proximales Ende des Schneidinstruments bildenden Griff- und Betätigungsteil umfasst.

Der Aufbau des Schneidinstruments lässt sich weiter vereinfachen, wenn der Griff- und Betätigungsteil zwei relativ zueinander um eine zweite Schwenkachse verschwenkbar gelagerte Betätigungsbranchen umfasst zum Bewegen der Schneidbacken von der Anlegestellung in die Schneidstellung. Durch entsprechendes Betätigen der beiden Betätigungsbranchen kann somit das Schneidgut schnell und einfach durchtrennt werden.

Ein konstruktiver Aufwand für das Schneidinstrument kann auf einfache Weise dadurch verringert werden, dass die erste und die zweite Schwenkachse parallel zueinander verlaufen. Denkbar ist es insbesondere, dass die erste und die zweite Schwenkachse identisch sind. Durch die parallele Ausrichtung der ersten und zweiten Schwenkachsen hat eine Bedienperson direkt eine taktile Rückmeldung über eine Bewegung der Schneiden in Abhängigkeit einer Bewegung der Betätigungsbranchen.

Um das Schneidinstrument besonders kompakt ausbilden zu können, ist es günstig, wenn die zwei Betätigungsbranchen direkt aneinander verschwenkbar gelagert sind. Ferner sind so auch keine weiteren Lagerungselemente erforderlich.

Grundsätzlich wäre es denkbar, die beiden Schwenkhebel jeweils unbeweglich mit einer Betätigungsbranche zu verbinden oder einstückig mit diesen auszubilden. Günstig ist es jedoch, wenn ein distales Ende jeder Betätigungsbranche mit einem proximalen Ende der beiden Schwenkhebel beweglich gekoppelt ist. So kann insbesondere jedem Schwenkhebel eine Betätigungsbranche zugeordnet sein. Durch die bewegliche Kopplung kann beispielsweise eine Kniehebelanordnung ausgebildet werden zwischen den Schwenkhebeln und den Betätigungsbranchen. Ferner können so auch gezielt und definiert Kraftübersetzungen oder -untersetzungen zum Übertragen von Betätigungskräften, die über die Betätigungsbranchen eingeleitet werden, auf die Schneiden.

Auf besonders einfache Weise lassen sich die Schwenkhebel und die Betätigungsbranchen miteinander verbinden, wenn sie relativ zueinander und/oder aneinander jeweils um eine Drehachse verdrehbar gelagert sind. Sie können so also relativ zueinander verschwenkt werden.

Um eine definierte Beweglichkeit der Schwenkhebel infolge einer Betätigung der Betätigungsbranchen sicherstellen zu können, ist es vorteilhaft, wenn die Drehachsen parallel zur zweiten Schwenkachse verlaufen. Insbesondere können sie auch parallel zur ersten Schwenkachse verlaufen.

Gemäß einer weiteren bevorzugten Ausführungsform der Erfindung kann es vorteilhaft sein, wenn zwischen den Betätigungsbranchen und den Schwenkhebeln jeweils ein Kniehebelgelenk ausgebildet ist. Dies gestattet es, wenn die Betätigungsbranchen gegeneinander verschwenkt werden, beispielsweise die Schneiden des Schneidinstruments automatisch etwas in proximaler Richtung zu bewegen, um eine zusätzliche Impulseinleitung in das Schneidgut in distaler Richtung zu verhindern oder zumindest zu verringern.

Der konstruktive Aufbau des Schneidinstruments kann sich weiter vereinfachen lassen, wenn die erste und die zweite Schwenkachse die Spiegelebene definieren.

Damit das Schneidinstrument vor einer Anwendung durch einen Operateur und nicht erst in die Anlagestellung überführt werden muss, ist es vorteilhaft, wenn eine Rückstelleinrichtung zum automatischen Rückführen des Schneidinstruments von der Schneidstellung in die Anlegestellung vorgesehen ist. Es nimmt dann, wenn es nicht betätigt wird, automatisch immer die Anlegestellung ein.

Grundsätzlich wäre es denkbar, die Rückstelleinrichtung so auszubilden, dass sie an den Schwenkhebeln angreift. Günstig ist es jedoch, wenn die Rückstelleinrichtung mit den beiden Betätigungsbranchen in Wirkverbindung steht. Eine Kraft zum Überführen des Schneidinstruments von der Schneidstellung in die Anlegestellung kann von der Rückstelleinrichtung auf diese Weise direkt in die Betätigungsbranchen eingeleitet werden.

Besonders einfach und kostengünstig herstellen lässt sich das Schneidinstrument, wenn die Rückstelleinrichtung mindestens ein Rückstellglied, insbesondere in Form eines Federelements, umfasst und wenn das Schneidinstrument entgegen einer vom mindestens einen Rückstellglied ausübbaren Rückstellkraft von der Anlegestellung in Schneidstellung bringbar ist. Optional kann die Rückstelleinrichtung auch derart ausgebildet sein, dass sie ausschaltbar ist. Die nachfolgende Beschreibung bevorzugter Ausführungsformen der Erfindung dient im Zusammenhang mit der Zeichnung der näheren Erläuterung. Es zeigen:
- Figur 1:: eine perspektivische Gesamtansicht eines chirurgischen Schneidinstruments beim Durchtrennen einer Sternum-Verschlussvorrichtung;

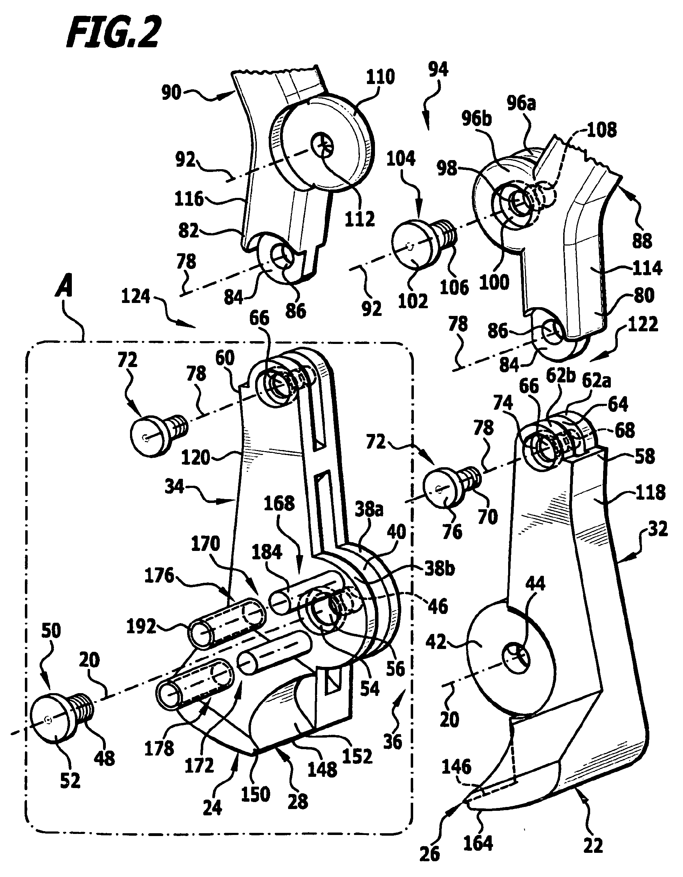

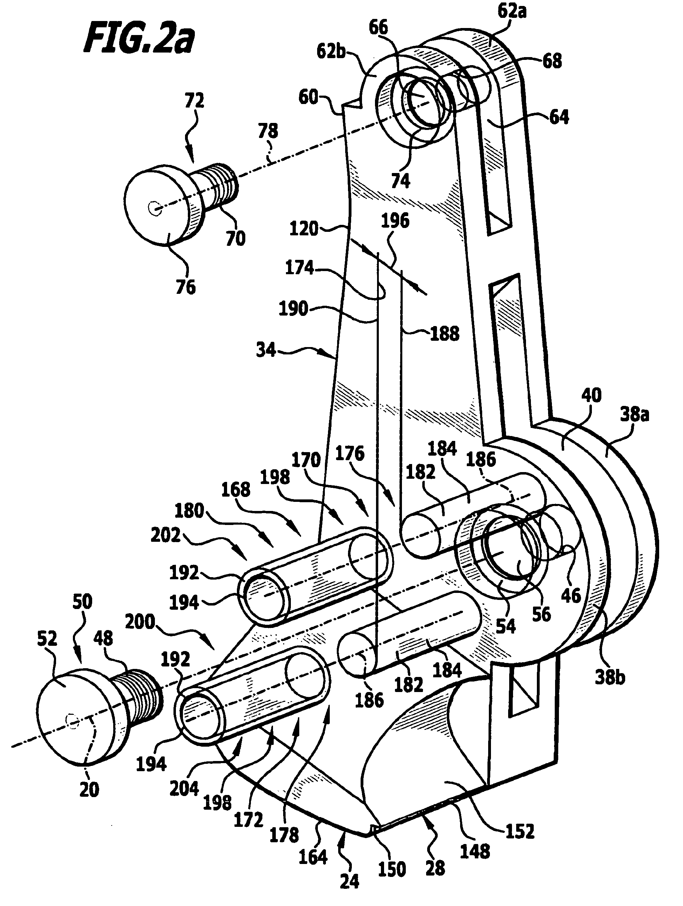

- Figur 2:: eine Explosionsdarstellung eines distalseitigen Teils des in Figur 1 dargstellten Schneidinstruments;
- Figur 2a:: eine vergrößerte Ansicht des Bereichs A in Figur 2;
- Figur 3:: eine Seitenansicht des in Figur 2 dargestellten Teils des Instruments in der Anlegestellung beim Anlegen an die Sternum-Verschlussvorrichtung;
- Figur 4:: eine Ansicht analog Figur 3 beim Anlegen der Schneidkanten an das Schneidgut;
- Figur 5:: eine Ansicht analog Figur 4 während des Durchtrennens des Schneidguts; und
- Figur 6:: eine Ansicht analog Figur 5 nach dem Durchtrennen des Schneidguts.

In den Figuren ist ein insgesamt mit dem Bezugszeichen 10 versehenes chirurgisches Schneidinstrument in Form eines Kopfschneiders dargestellt. Es dient insbesondere dem Zweck, stiftförmige Verbindungselemente 12 einer Sternumverschlussvorrichtung 14 zu durchtrennen, und zwar einen nach Anlegen und Fixieren der Sternumverschlussvorrichtung 14 an ein eröffnetes Sternum 15 überstehenden Teil 16 der Verbindungselemente 12. Der prinzipielle Aufbau der Sternumverschlussvorrichtung 14 ist im Einzelnen in der DE 103 26 690 B4 beschrieben, eine Applikation derselben mittels eines speziell dafür ausgestalteten Anlegeinstruments ist in der DE 20 2007 007 880 U1 offenbart. Beide Veröffentlichungen werden mit ihrem gesamten Offenbarungsgehalt hiermit in die vorliegende Anmeldung mit einbezogen. Die Sternumverschlussvorrichtung 14, insbesondere die Verbindungselemente 12 bilden ein mit dem Schneidinstrument 10 durchtrennbares Schneidgut 18.

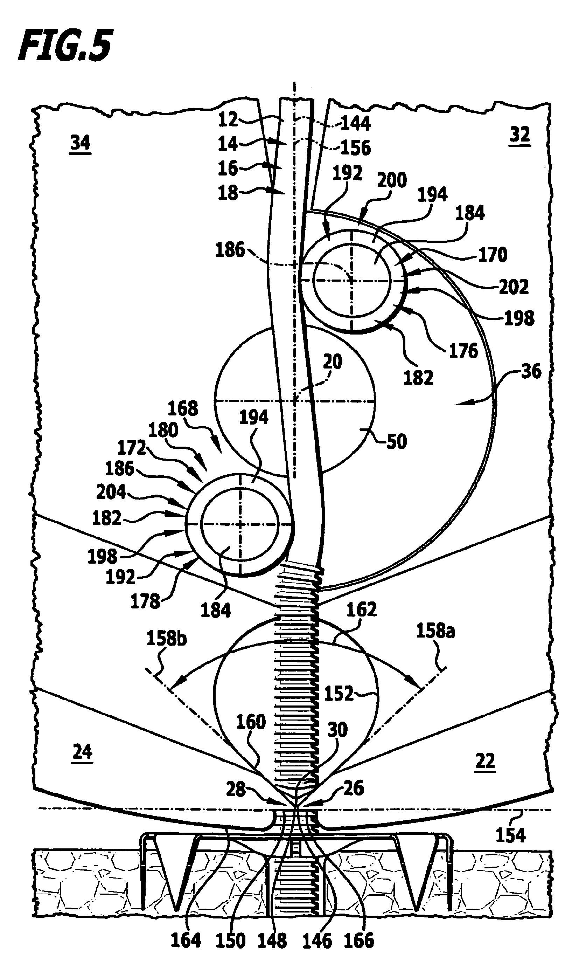

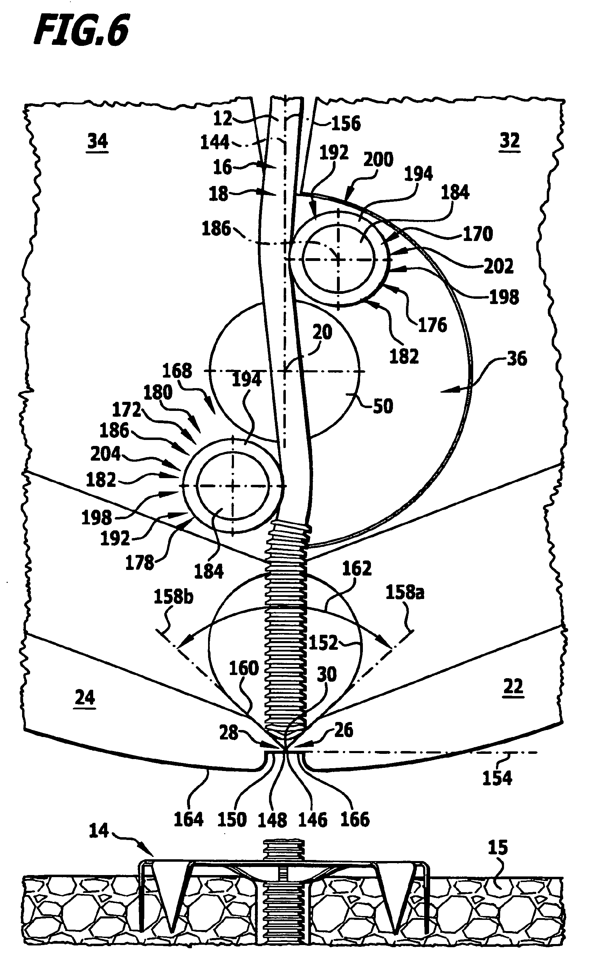

Das Schneidinstrument 10 umfasst zwei um eine erste Schwenkachse 20 relativ zueinander verschwenkbare Schneidbacken 22 und 24, die jeweils eine Schneide 26 beziehungsweise 28 umfassen, die in einer Schneidstellung, wie sie in den Figuren 5 und 6 dargestellt ist, längs einer Schneidlinie 30 aneinander anliegen und im Wesentlichen aneinander anliegen zum Durchtrennen des Schneidguts 18. Die Schneidbacken 22 und 24 sind als seitlich abstehende Vorsprünge an einem distalen Ende jeweils eines im Wesentlichen quaderförmigen Schwenkhebels 32 beziehungsweise 34 angeformt.

Ein Schwenklager 36 dient zur verschwenkbaren Lagerung der Schwenkhebel 32 und 34 aneinander um die erste Schwenkachse 20. Das Schwenklager 36 umfasst zwei voneinander beabstandete Lagerbacken 38, die zwischen sich einen Spalt 40 definieren. Die Lagerbacken 38 sind am Schwenkhebel 34 ausgebildet als Vorsprünge in Form von halben Scheiben, die in Richtung auf den Schwenkhebel 32 hin weisen. Der Spalt 40 ist gerade so groß ausgebildet, dass er eine Lagerscheibe 42, die am Schwenkhebel 32 in Richtung auf den Schwenkhebel 34 hin weisend abstehend ausgebildet ist, aufnehmen kann.

Die Lagerscheibe 42 ist koaxial zur Schwenkachse 20 mit einer Bohrung 44 versehen. Die Lagerbacken 38a und 38b sind ebenfalls durchbohrt. Ein den Lagerbacken 38a durchsetzender Abschnitt einer Bohrung 56 ist mit einem Innengewinde 46 versehen, welches zu einem Außengewinde 48 eines zylindrischen Schraubbolzens 50 ausgebildet ist, welcher einen scheibenförmigen Kopf 52 aufweist. An einem Abschnitt der Bohrung 56, die den Lagerbacken 38b durchsetzt, ist ein Rücksprung 54 ausgebildet, welcher die Bohrung 56 im Innendurchmesser einstufig und von der Lagerbacke 38a weg weisend erweitert. Der Rücksprung 54 ist so dimensioniert, dass der Kopf 52 formschlüssig aufgenommen werden kann. Der Abschnitt der Bohrung 56 an der Lagerbacke 38b weist einen Außendurchmesser auf, der es gestattet, dass der das Außengewinde 48 tragende Bolzenabschnitt des Schraubbolzens 50 durchsteckbar ist. Die Bohrung 44 weist einen Innendurchmesser auf, der einem Außendurchmesser des Außengewindes 48 entspricht.

Zur Montage der Schenkhebel 32 und 34 aneinander wird die Lagerscheibe 42 in den Spalt 40 eingeführt, der Schraubbolzen 50 zuerst durch die Bohrung 56 an der Lagerbacke 38b und dann durch die Bohrung 44. Mittels des Außengewindes 48 wird der Schraubbolzen mit dem Innengewinde 46 an der Lagerbacke 38a verschraubt und sichert so die beiden Schwenkhebel 32 und 34 verschwenkbar aneinander.

Proximale Enden 58 und 60 der Schwenkhebel 32 und 34 weisen jeweils zwei weitere Lagerbacken 62a und 62b auf, die zwischen sich einen Schlitz 64 definieren. Die Lagerbacken 62a und 62b sind in Form halber Scheiben ausgebildet und weisen im Wesentlichen in proximaler Richtung. Die Lagerbacken 62a und 62b sind jeweils mit einer Bohrung 66 versehen, wobei die Lagerbacken 62a durchsetzende Abschnitte der Bohrungen 66 jeweils mit einem Innengewinde 68 versehen sind, welche zu einem Außengewinde 70 eines Schraubbolzens 72 korrespondierend ausgebildet ist. Im Bereich des Lagerbackens 62b ist ein Innendurchmesser der Bohrung 66 durch einen Rücksprung 74 vom Lagerbacken 62a weg weisend einstufig erweitert. Der Rücksprung 74 ist korrespondierend ausgebildet zu einem Kopf 76 des Schraubbolzens 72, der so formschlüssig aufgenommen werden kann.

Längsachsen der Bohrungen 66 definieren Drehachsen 78, die parallel zur ersten Schwenkachse 20 verlaufen. Die Schlitze 64 sind ausgebildet zur Aufnahme jeweils einer ein distales Ende 80 beziehungsweise 82 zweier Betätigungsbranchen 88 und 90 definierenden Lagerscheibe 84, die ebenfalls mit einer Bohrung 86 versehen ist, deren Innendurchmesser einem Außendurchmesser des das Außengewinde 70 tragenden Bolzenabschnitts des Schraub-bolzens 72 entspricht. Die Betätigungsbranchen 88 und 90 sind um eine zweite Schwenkachse 92 verschwenkbar aneinander gelagert, und zwar mittels eines entsprechend dem Schwenklager 36 ausgebildeten Schwenklagers 94.

Die proximalen Enden 58 und 60 sind mit den distalen Enden 80 und 82 verbunden durch Einführen der Lagerscheiben 84 in die Schlitze 64 und anschließendes Einführen der Schraubbolzen 72 durch die Lagerbacken 62b und Verschrauben der Außengewinde 70 mit den Innengewinden 68 der Lagerbacken 62a.

Etwas beabstandet vom Ende 80 sind an der Betätigungsbranche 88 zwei im Wesentlichen in Form halber Scheiben in Richtung auf die Betätigungsbranche 90 abstehende Vorsprünge ausgebildet, die Lagerbacken 96a und 96b definieren welche von einer Bohrung 98 durchsetzt sind. Ein Abschnitt der Bohrung 98 ist in der Lagerbacke 96b durch einen Rücksprung 100 im Innendurchmesser einstufig erweitert, so dass sie den Kopf 102 eines mit dem Schraubbolzen 50 identisch ausgebildeten Schraubbolzens 104 formschlüssig aufnehmen kann, wenn ein Außengewinde 106 durch die Bohrung 98 in der Lagerbacke 96b durchgesteckt und mit einem in einen Abschnitt der Bohrung 98 an der Lagerbacke 96a ausgebildeten Innengewinde 108 verschraubt wird. Auf dem das Außengewinde 106 tragenden Bolzenabschnitt des Schraubbolzens 104 ist eine Lagerscheibe 110 um die zweite Schwenkachse 92 zwischen den Lagerbacken 96a und 96b verschwenkbar gelagert, welche eine konzentrisch zur zweiten Schenkachse 92 ausgebildete Bohrung 112 aufweist, deren Innendurchmesser dem Außendurchmesser des Außengewindes 106 entspricht.

Die Betätigungsbranchen 88 und 90 definieren zwei sich bezogen auf die zweite Schwenkachse 92 in distaler Richtung erstreckende Hebelabschnitte 114 und 116, welche mit sich in proximaler Richtung von der ersten Schwenkachse 20 an den Schwenkhebeln 32 und 34 definierten Hebelabschnitten 118 und 120 in der beschriebenen Weise verschwenkbar gekoppelt sind. Insgesamt werden so zwei Kniehebelgelenke 122 und 124 ausgebildet durch die um die Drehachsen 78 verschwenkbar aneinander gelagerten Hebelabschnitte 114 und 118 sowie 116 und 120.

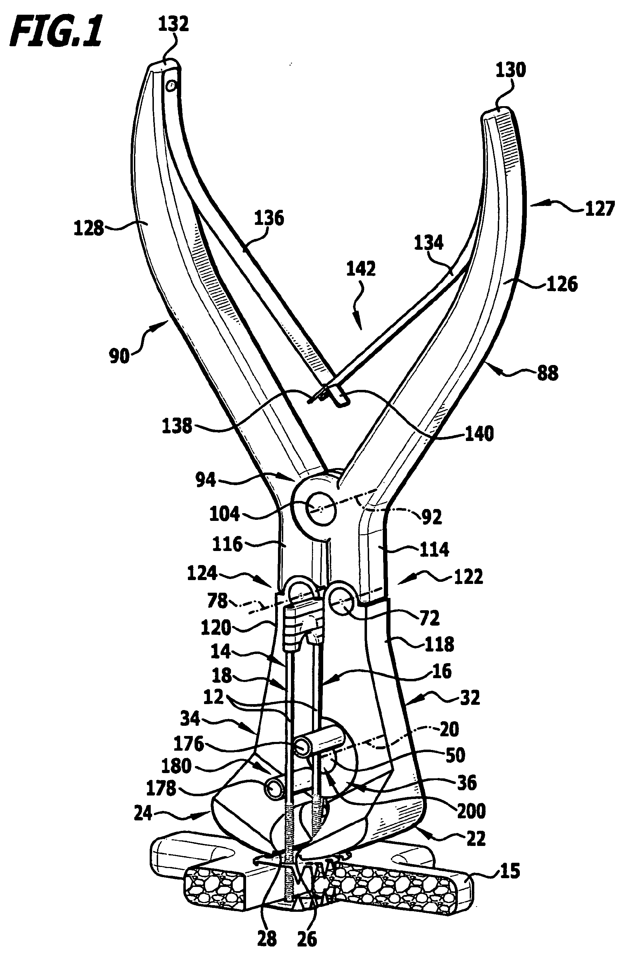

Proximalseitig der zweiten Schwenkachse 92 weisen die Betätigungsbranchen 88 und 90 geschwungene Betätigungsabschnitte 126 und 128 auf. Die Betätigungsabschnitte 126 und 128 bilden so einen Griff- und Betätigungsteil 127 aus, welcher ein proximales Ende des Schneidinstruments 10 bildet.
An freien Enden 130 und 132 der Betätigungsbranchen 88 und 90 sind jeweils freie Enden eines Rückstellglieds 134 beziehungsweise 136 in Form einer Blattfeder festgelegt. Freie, in distaler Richtung weisende Enden 138 und 140 der Rückstellglieder 134 und 136, stehen zwischen den Betätigungsabschnitten 126 und 128 etwas proximalseitig der zweiten Schwenkachse 92 miteinander in Eingriff. Die Rückstellglieder 134 und 136 bilden eine insgesamt mit dem Bezugszeichen 142 bezeichnete Rückstelleinrichtung aus zum automatischen Überführen des Schneidinstruments 10 von der Schneidstellung in die Anlegestellung. Das Schneidinstrument 10 kann so entgegen einer von den zusammenwirkenden Rückstellgliedern 134 und 136 ausgeübten Rückstellkraft von der Anlegestellung, wie sie beispielsweise in Figur 1 dargestellt ist, in die Schneidstellung, wie sie in den Figuren 5 und 6 dargestellt ist, überführt werden durch Verschwenken der Enden 130 und 132 aufeinander zu.

Die erste Schwenkachse 20 und die zweite Schwenkachse 92 definieren eine Spiegelebene 144 bezüglich derer die Schneidbacken 24 und 26 spiegelsymmetrisch ausgebildet sind.

Jede der Schneiden 26 und 28 weist eine Schneidkante 146 beziehungsweise 148 auf, die in einer Schneidstellung des Schneidinstruments 10 linienförmig oder im Wesentlichen linienförmig aneinander anliegen und die Schneidlinie 30 definieren. Jede Schneide 26, 28 weist eine erste, in distaler Richtung weisende Seitenfläche 150 sowie eine zweite, im Wesentlichen in proximaler Richtung weisende Seitenfläche 152 auf. Die ersten Seitenflächen 150 definieren eine erste Schneidebene 154, welche in der Schneidstellung, wie in Figur 5 dargestellt, eine vom Schneidinstrument 10 definierte Längsachse 156, die in der Spiegelebene 144 liegt, senkrecht schneidet. Die zweiten Seitenflächen 152 definieren zweite Schneidebenen 158a und 158b, welche in der Schneidstellung relativ zu der vom Schneidinstrument 10 definierten Längsachse 156 geneigt sind. Die beiden zweiten Seitenflächen 152 der Schneiden 26 und 28 begrenzen in der Schneidstellung eine in proximaler Richtung weisende keilförmige Vertiefung 160, welche einen Öffnungswinkel 162 definiert. Der Öffnungswinkel 162 liegt vorzugsweise in einem Bereich von 30° bis 160°. Günstig ist es, wenn er in einem Bereich von 60° bis 120° liegt. Bei dem in den Figuren dargestellten Ausführungsbeispiel beträgt der Öffnungswinkel 162 etwa 90°.

Ein distales Ende 164 des Schneidinstruments 10 weist in der Schneidstellung eine nutförmige Ausnehmung 166 auf. Die ersten Seitenflächen 150 bilden einen Nutboden, welcher in distaler Richtung weist und definieren beziehungsweise begrenzen die Ausnehmung 166 somit teilweise. Die Schneidkanten 146 und 148 sind durch die Ausbildung der Ausnehmung 166 vom distalen Ende 164 axial, das heißt parallel zur Längsachse 156, beabstandet.

Das Schneidinstrument 10 umfasst ferner eine insgesamt mit dem Bezugszeichen 168 bezeichnete Halteeinrichtung 168 zum Halten des abzutrennenden beziehungsweise abgetrennten Teils 16 des Schneidguts 18 vor, während und nach dem Durchtrennen desselben. Die Halteeinrichtung 168 umfasst zwei Halteglieder 170 und 172, die zwischen sich eine Schneidgutaufnahme 174 definieren. Die Halteglieder 170 und 172 bilden gleichzeitig Verformungselemente 176 und 178 einer insgesamt mit dem Bezugszeichen 180 bezeichneten Verformungseinrichtung.

Die Verformungselemente 176 und 178 umfassen jeweils ein Lagerglied 182 in Form eines zylindrischen Lagerbolzens 184. Beide Lagerglieder 182 sind mit ihren Rotationsachsen 186 definierenden Verformungselementlängsachsen parallel zur ersten Schwenkachse 20 von der Lagerbacke 38b abstehend angeordnet. Die Lagerbolzen 184 sind ferner bezogen auf die erste Schwenkachse 20 einander diametral gegenüberliegend und jeweils auf einer Seite der Spiegelebene 144 angeordnet. Das erste Verformungselement 176 ist vom distalen Ende 164 des Schneidinstruments 10 weiter beabstandet als das zweite Verformungselement 178. Die Verformungselemente 176 und 178 sind somit zur Spiegelebene 144 unsymmetrisch angeordnet.

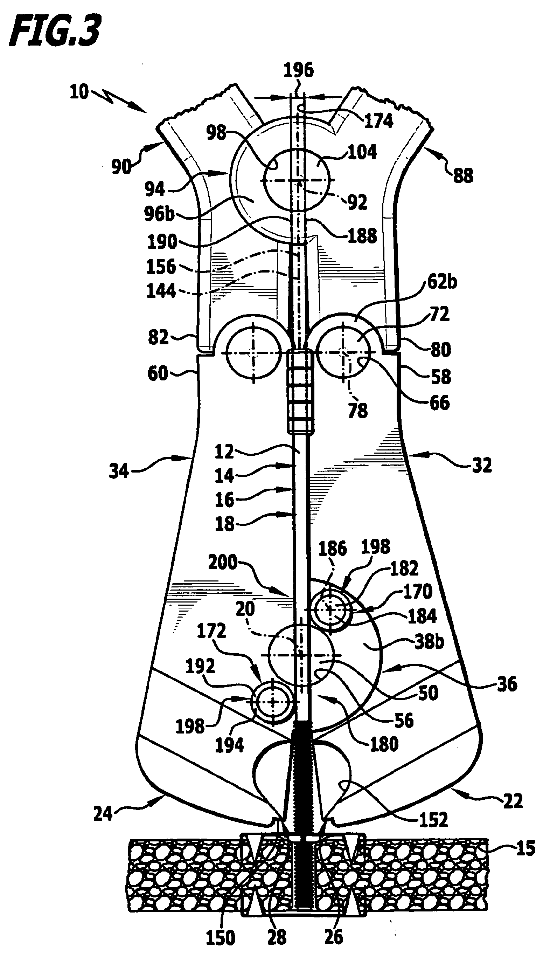

Die Schneidgutaufnahme 174 wird definiert und begrenzt durch zwei zueinander parallele Schneidgutaufnahmebenen 188 und 190, die parallel zur Spiegelebene 144 und auf unterschiedlichen Seiten derselben verlaufen. In der in Figur 3 dargestellten Anlegestellung berühren die Verformungselemente 176 und 178 die Schneidgutaufnahme 174 entlang der die Schneidgutaufnahme 174 begrenzenden Schneidgutaufnahmeebenen 188 und 190. Damit begrenzen die Verformungselemente 176 und 178 die Schneidgutaufnahme 174 zumindest teilweise.

Die Verformungselemente 176 und 178 umfassen ferner jeweils ein Verformungsglied 192 in Form einer Lagerhülse 194, welche um die Verformungselementlängsachse rotierbar am Lagerbolzen 184 gelagert sind.

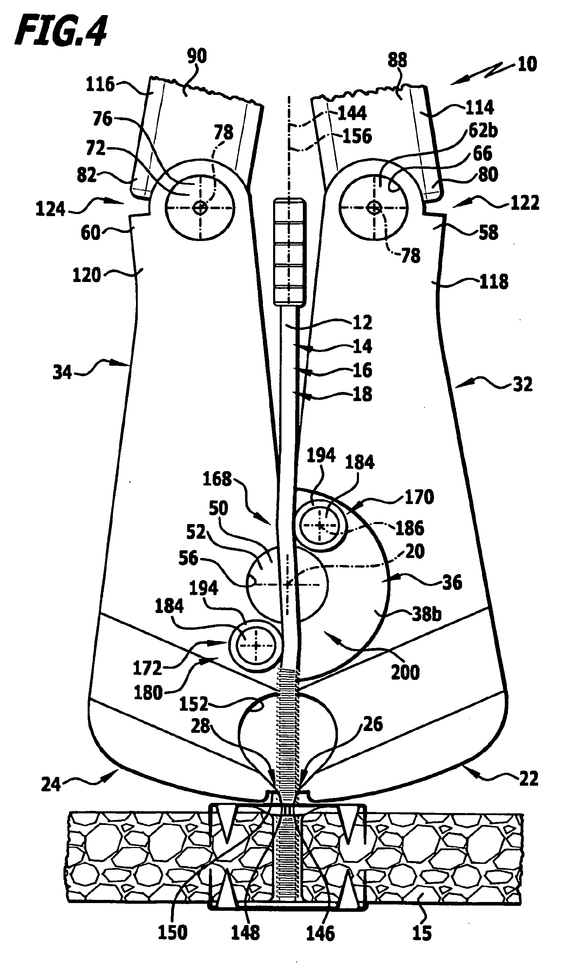

Werden die Schwenkhebel 32 und 34 relativ zueinander um die erste Schenkachse 20 verschwenkt, bewegt sich das Verformungselement 176 in Richtung auf die Spiegelebene 144 hin, ebenso das Verformungselement 178, jedoch von der anderen Seite. Damit ändert sich auch ein Abstand der Schneidgutaufnahmeebenen 188 und 190 voneinander oder mit anderen Worten, eine Breite 196 der Schneidgutaufnahme 174. Die Breite 196 weist einen maximalen Wert auf in der in Figur 3 dargestellten Anlegestellung, so dass die Verbindungselemente 12 parallel zur Längsachse 156 in die Schneidgutaufnahme 174 einführbar sind. Beim Überführen des Schneidinstruments 10 von der Anlegestellung in die Schneidstellung, werden die Verbindungselemente 12, die an den Verformungselementen 176 und 178 anliegen sowie zwischen den Schneiden 26 und 28 gehalten sind, in Folge der Verschwenkbewegung der Verformungselemente 176 und 178 um die erste Schwenkachse 20 schwach s-förmig verformt, wie dies in den Figuren 4 bis 6 dargestellt ist. Es wird so eine Dreipunktauflage definiert durch die beiden Verformungselemente 176 und 178 sowie die beiden die Verbindungselemente 12, also das Schneidgut 18, zwischen sich klemmenden Schneiden 26 und 28.

In der Schneidstellung tauchen die Verformungselemente 176 und 178 zumindest teilweise in die in der Anlagestellung definierte Schneidgutaufnahme 174 ein, in der die Schneidgutaufnahme 174, wie sie in Figur 3 dargestellt ist, eine maximale Breite 196 aufweist. In der Schneidstellung ist die Schneidgutaufnahme 174 folglich so schmal, dass ihre Breite 196 kleiner ist als eine Dicke des Schneidguts 18, so dass es zwangsläufig zu der beschriebenen und gewünschten Verformung und Verklemmung des Schneidguts kommt.

Die oben beschriebenen Verformungselemente 176 und 178 sind also in Form jeweils eines Haltebolzens 198 ausgebildet, welcher eine Haltebolzenlängsachse definiert, die der Rotationsachse 186 entspricht, welche parallel oder im Wesentlichen parallel zur Schneidlinie 30 verläuft.

Das Schneidinstrument 10 umfasst ferner eine insgesamt mit dem Bezugszeichen 200 bezeichnete Schneidimpulsaufnahmeeinrichtung zum Aufnehmen eines beim Durchtrennen des auf den abgetrennten Teil 16 des Schneidguts 18 wirkenden Schneidimpulses. Sie umfasst zwei Impulsaufnahmeelemente 202 und 204, die bewegbar am Schneidinstrument 10 gelagert sind und die Schneidgutaufnahme 174 zumindest teilweise begrenzen. Sie werden beim Schneidinstrument 10, welches in den Figuren dargestellt ist, gebildet durch die Verformungselemente 176 beziehungsweise 178. Die Impulsaufnahmeelemente 202 und 204 sind folglich in Folge einer Bewegung des vom Schneidgut 18 abzutrennenden Teils 16 in proximaler Richtung bewegbar, nämlich um die Rotationsachsen 186 rotierbar.

Beim Schneidvorgang werden die keilförmigen Schneiden 26 und 28 von beiden Seiten in das Schneidgut 18 getrieben, so dass dieses zunächst etwas voneinander getrennt wird, wie in den Figuren 4 und 5 dargestellt. Mit fortschreitendem Eindringen der Schneiden 26 und 28 in das Schneidgut 18 werden durch die zweiten Seitenflächen 152 der Schneiden 26 und 28 Kräfte entwickelt, die nicht nur in Schneidrichtung, also quer zur Längsachse 156, wirken, sondern auch parallel zur Längsachse 156 in proximaler Richtung. Aufgrund dieser Kräfte wird das Schneidgut 18 geringfügig in einer Richtung parallel zur Längsachse 156 gedehnt und legt dabei einen Weg s zurück. Die rotierbaren Verformungsglieder 192 leiten diese Längendehnung direkt, das heißt 1:1, weiter, so dass beim Schneidinstrument 10 kein Kraftstau aufgrund der großen Reibung an der Halteeinrichtung 168 entstehen kann, der sich beim endgültigen Durchtrennen des Schneidguts 18, wie bei herkömmlichen Schneidinstrumenten, plötzlich entladen und den Teil 16 wegsprengen würde. Die nach dem Durchtrennen noch wirkenden Absprengkräfte, das heißt der Schneidimpuls, werden durch die wie in den Figuren 4 bis 6 gut zu erkennen, s-förmige Verspannung oder Verformung des Schneidguts 18 mittels der Verformungseinrichtung 180 sicher kompensiert. Der abgetrennte Teil 16 bleibt weiter an der Halteeinrichtung 168 in der Schneidgutaufnahme 174 gehalten.

Ferner wird bei dem Schneidinstrument 10 nur ein Schneidimpuls erzeugt, dessen Kraftrichtung in proximaler Richtung und damit vom Sternum 15 weg verläuft. Dies wird erreicht durch die spezielle Schneidengeometrie der Schneiden 26 und 28, nämlich dadurch, dass die erste Schneidebene 154 senkrecht zur Längsachse 156 verläuft. So kann kein Impuls in Richtung auf das Sternum 15 wirken, welcher die Sternumverbindungsvorrichtung 14, insbesondere die Verbindungselemente 12, in Richtung auf das Körperinnere treiben und/oder die Sternumverschlussvorrichtung 14 lösen könnte.

Aufgrund der rotierbaren Anordnung der Verformungsglieder 192 ist die Schneidimpulsaufnahmeeinrichtung 200 praktisch verschleißfrei, so dass das Schneidinstrument 10, solange die Schneiden 26 und 28 scharf genug sind, zum sicheren und rückstoßfreien Durchtrennen eines Schneidguts 18 eingesetzt werden kann.

## Patentansprüche

1. Chirurgisches Schneidinstrument (10) zum Durchtrennen eines insbesondere stiftförmigen Schneidguts (18), welches Schneidinstrument (10) zwei relativ zueinander bewegbare, jeweils eine Schneide (26, 28) aufweisende Schneidbacken (22, 24) umfasst, welche Schneiden (26, 28) in einer Anlegestellung voneinander beabstandet sind und in einer Schneidstellung längs einer Schneidlinie (30) aneinander anliegen oder im Wesentlichen aneinander anliegen zum Durchtrennen des Schneidguts (18), wobei das Schneidinstrument (10) eine Halteeinrichtung (168) zum Halten eines abgetrennten Teils (16) des Schneidguts (18) nach dem Durchtrennen desselben umfasst, wobei die Halteeinrichtung (168) eine Schneidgutaufnahme für einen abzutrennenden Teil des Schneidguts aufweist und wobei die Halteeinrichtung (168) mindestens ein Halteglied (170, 172) zum Halten des abzutrennenden Teils (16) in der Schneidgutaufnahme (174) vor und/oder nach dem Durchtrennen des Schneidguts (18) umfasst, wobei eine Verformungseinrichtung (180) zum Verformen des in der Schneidgutaufnahme (174) gehaltenen und vom Schneidgut (18) abzutrennenden Teils (16) desselben vorgesehen ist und wobei die Verformungseinrichtung (180) mindestens ein Verformungselement (176, 178) umfasst, welches relativ zur Schneidgutaufnahme (174) beim Übergang von der Anlegestellung in die Schneidstellung bewegbar ist und wobei das mindestens eine Verformungselement (176, 178) mindestens teilweise in die in der Anlegestellung definierte Schneidgutaufnahme (174) hineinbewegbar ist beim Übergang von der Anlegestellung in die Schneidstellung, **dadurch gekennzeichnet, dass** mindestens ein Teil (192) des mindestens einen Verformungselements (176, 178) um eine Verformungselementlängsachse rotierbar gelagert ist.

2. Schneidinstrument nach Anspruch 1, **dadurch gekennzeichnet, dass** das mindestens eine Verformungselement (176, 178) die Schneidgutaufnahme (174) mindestens teilweise begrenzt.

3. Schneidinstrument nach Anspruch 1 oder 2, **dadurch gekennzeichnet, dass** ein erstes Verformungselement (176) von einem distalen Ende (164) des Schneidinstruments (10) weiter beabstandet ist als ein zweites Verformungselement (178).

4. Schneidinstrument nach einem der voranstehenden Ansprüche, **dadurch gekennzeichnet, dass** das mindestens eine Verformungselement (176, 178) in Form eines Haltebolzens (198) ausgebildet ist.

5. Schneidinstrument nach einem der voranstehenden Ansprüche, **dadurch gekennzeichnet, dass** das mindestens eine Verformungselement (176, 178) als Ganzes rotierbar um die Verformungselementlängsachse (186) gelagert ist.

6. Schneidinstrument nach einem der voranstehenden Ansprüche, **dadurch gekennzeichnet, dass** das mindestens eine Verformungselement (176, 178) ein Lagerglied (182) und ein Verformungsglied (192) umfasst und dass das Verformungsglied (192) am Lagerglied (182) um die Verformungselementlängsachse (186) rotierbar gelagert ist.

7. Schneidinstrument nach Anspruch 6, **dadurch gekennzeichnet, dass** das Lagerglied (182) in Form eines zylindrischen Lagerbolzens (184) ausgebildet ist und dass das Verformungsglied (192) in Form einer auf dem Lagerbolzen (184) rotierbar gelagerten Verformungshülse (194) ausgebildet ist.

8. Schneidinstrument nach Anspruch 6 oder 7, **dadurch gekennzeichnet, dass** die Schneidbacken (22, 24) relativ zueinander um eine erste Schwenkachse (20) verschwenkbar angeordnet oder gelagert sind, dass das mindestens eine Verformungselement (176, 178) um die erste Schwenkachse (20) verschwenkbar gelagert ist, dass zwei um die erste Schwenkachse (20) verschwenkbar aneinander gelagerte Schwenkhebel (32, 34) vorgesehen sind, an denen jeweils eine Schneidbacke (22, 24) angeordnet oder ausgebildet ist, und dass das mindestens eine Verformungselement (176, 178) an den Schwenkhebeln (34) angeordnet oder ausgebildet ist.

9. Schneidinstrument nach einem der voranstehenden Ansprüche, **gekennzeichnet durch** eine Schneidimpulsaufnahmeeinrichtung (200) zum Aufnehmen eines beim Durchtrennen zumindest auf einen abgetrennten Teil (16) des Schneidguts (18) wirkenden Schneidimpulses, wobei die Schneidimpulsaufnahmeeinrichtung (200) mindestens ein Impulsaufnahmeelement (202, 204) aufweist, welches bewegbar am Schneidinstrument (10) gelagert ist und die Schneidgutaufnahme (174) mindestens teilweise begrenzt.

10. Schneidinstrument nach einem der voranstehenden Ansprüche, **dadurch gekennzeichnet, dass** jede Schneide (26, 28) eine erste, in distaler oder im Wesentlichen in distaler Richtung weisende Seitenfläche (150) und eine zweite, in proximaler oder im Wesentlichen in proximaler Richtung weisende Seitenfläche (152) aufweist.

11. Schneidinstrument nach Anspruch 10, **dadurch gekennzeichnet, dass** die erste Seitenfläche (150) eine erste Schneidebene (154) definiert, welche in der Schneidstellung eine vom Schneidinstrument (10) definierte Längsachse (156) senkrecht oder im Wesentlichen senkrecht schneidet.

12. Schneidinstrument nach Anspruch 10 oder 11, **dadurch gekennzeichnet, dass** die zweite Seitenfläche (152) eine zweite Schneidebene (158a, 158b) definiert, welche in der Schneidstellung relativ zu einer vom Schneidinstrument (10) definierten Längsachse geneigt ist.

13. Schneidinstrument nach einem der Ansprüche 10 bis 12, **dadurch gekennzeichnet, dass** die beiden zweiten Seitenflächen (152) der beiden Schneiden (26, 28) in der Schneidstellung eine in proximaler Richtung weisende keilförmige Vertiefung (160) begrenzen und dass die Vertiefung (160) einen Öffnungswinkel (162) definiert.

14. Schneidinstrument nach einem der Ansprüche 10 bis 13, **dadurch gekennzeichnet, dass** ein distales Ende (164) des Schneidinstruments (10) in der Schneidstellung eine nutförmige Ausnehmung (166) aufweist und dass die ersten oder zweiten Schneidflächen die Ausnehmung mindestens teilweise definieren oder begrenzen.

15. Schneidinstrument nach einem der voranstehenden Ansprüche, **gekennzeichnet durch** einen ein proximales Ende des Schneidinstruments (10) bildenden Griff- und Betätigungsteil (127), und dass der Griff- und Betätigungsteil (127) zwei relativ zueinander um eine zweite Schwenkachse (92) verschwenkbar gelagerte Betätigungsbranchen (88, 90) umfasst zum Bewegen der Schneidbacken (22, 24) von der Anlegestellung in die Schneidstellung.

## Claims

1. Surgical cutting instrument (10) for severing an, in particular, pinshaped material to be cut (18), which cutting instrument (10) comprises two cutting jaws (22, 24) which are movable relative to each other and have one cutter (26, 28) each, which cutters (26, 28) are spaced from each other in an applying position, and lie against each other or lie substantially against each other along a cutting line (30) in a cutting position for severing the material to be cut (18), the cutting instrument (10) comprising a holding device (168) for holding a cut-off part (16) of the material to be cut (18) after the severing thereof, the holding device (168) comprising a receptacle for material to be cut for a part that is to be cut off from the material to be cut, and the holding device (168) having at least one holding member (170, 172) for holding the part to be cut off (16) in the receptacle for material to be cut (174) before and/or after the severing of the material to be cut (18), a deformation device (180) being provided for deforming the part (16) that is held in the receptacle for material to be cut (174) and is to be cut off the material to be cut (18), and the deformation device (180) comprising at least one deformation element (176, 178) which is movable relative to the receptacle for material to be cut (174) during the transition from the applying position to the cutting position, and the at least one deformation element (176, 178) being movable at least partially into the receptacle for material to be cut (174) defined in the applying position during the transition from the applying position to the cutting position, **characterized in that** at least one part (192) of the at least one deformation element (176, 178) is mounted for rotation about a longitudinal axis of the deformation element.

2. Cutting instrument in accordance with claim 1, **characterized in that** the at least one deformation element (176, 178) delimits the receptacle for material to be cut (174) at least partially.

3. Cutting instrument in accordance with claim 1 or 2, **characterized in that** a first deformation element (176) is spaced further from a distal end (164) of the cutting instrument (10) than is a second deformation element (178).

4. Cutting instrument in accordance with any one of the preceding claims, **characterized in that** the at least one deformation element (176, 178) is configured in the form of a holding bolt (198).

5. Cutting instrument in accordance with any one of the preceding claims, **characterized in that** the at least one deformation element (176, 178) is mounted in its entirety for rotation about the longitudinal axis (186) of the deformation element.

6. Cutting instrument in accordance with any one of the preceding claims, **characterized in that** the at least one deformation element (176, 178) comprises a bearing member (182) and a deformation member (192), and **in that** the deformation member (192) is mounted on the bearing member (182) for rotation about the longitudinal axis (186) of the deformation element.

7. Cutting instrument in accordance with claim 6, **characterized in that** the bearing member (182) is configured in the form of a cylindrical bearing bolt (184), and **in that** the deformation member (192) is configured in the form of a deformation sleeve (194) mounted for rotation on the bearing bolt (184).

8. Cutting instrument in accordance with claim 6 or 7, **characterized in that** the cutting jaws (22, 24) are arranged or mounted for pivotal movement relative to each other about a first pivot axis (20), **in that** the at least one deformation element (176, 178) is mounted for pivotal movement about the first pivot axis (20), **in that** two pivot levers (32, 34) are provided, which are mounted on each other for pivotal movement about the first pivot axis (20), and on each of which a cutting jaw (22, 24) is arranged or formed, and **in that** the at least one deformation element (176, 178) is arranged or formed on the pivot levers (34).

9. Cutting instrument in accordance with any one of the preceding claims, **characterized by** a cutting pulse receiving device (200) for receiving a cutting pulse acting during the severing at least on a cut-off part (16) of the material to be cut (18), the cutting pulse receiving device (200) comprising at least one pulse receiving element (202, 204), which is mounted for movement on the cutting instrument (10) and which delimits the receptacle for material to be cut (174) at least partially.

10. Cutting instrument in accordance with any one of the preceding claims, **characterized in that** each cutter (26, 28) comprises a first side face (150) pointing in the distal or substantially in the distal direction and a second side face (152) pointing in the proximal or substantially in the proximal direction.

11. Cutting instrument in accordance with claim 10, **characterized in that** the first side face (150) defines a first cutting plane (154) which, in the cutting position, intersects perpendicularly or substantially perpendicularly a longitudinal axis (156) defined by the cutting instrument (10).

12. Cutting instrument in accordance with claim 10 or 11, **characterized in that** the second side face (152) defines a second cutting plane (158a, 158b) which, in the cutting position, is inclined relative to a longitudinal axis defined by the cutting instrument (10).

13. Cutting instrument in accordance with any one of claims 10 to 12, **characterized in that** the two second side faces (152) of the two cutters (26, 28) delimit, in the cutting position, a wedge-shaped depression (160) pointing in the proximal direction, and **in that** the depression (160) defines an angle of aperture (162).

14. Cutting instrument in accordance with any one of claims 10 to 13, **characterized in that** a distal end (164) of the cutting instrument (10) comprises, in the cutting position, a groove-shaped recess (166), and **in that** the first or second cutting faces define or delimit the recess at least partially.

15. Cutting instrument in accordance with any one of the preceding claims, **characterized by** a gripping and actuating part (127) forming a proximal end of the cutting instrument (10), and in that the gripping and actuating part (127) comprises two actuating handles (88, 90) mounted for pivotal movement about a second pivot axis (92) relative to each other for moving the cutting jaws (22, 24) from the applying position to the cutting position.

## Revendications

1. Instrument de coupe chirurgical (10) destiné à sectionner un élément objet de la coupe (18), notamment en forme de tige, ledit instrument de coupe (10) comprenant deux mâchoires de coupe (22, 24) mobiles l'une par rapport à l'autre, pourvues chacune d'un tranchant (26, 28), lesdits tranchants (26, 28) étant espacés l'un de l'autre dans une position de repos et reposant l'un contre l'autre le long d'une ligne de coupe (30) dans une position de coupe, ou reposant sensiblement l'un contre l'autre pour sectionner l'élément objet de la coupe (18), l'instrument de coupe (10) comprenant un dispositif de maintien (168) pour le maintien d'une partie sectionnée (16) de l'élément objet de la coupe (18) après le sectionnement de celui-ci, le dispositif de maintien (168) comportant une réception d'élément objet de la coupe pour une partie à sectionner de l'élément objet de la coupe et le dispositif de maintien (168) comprenant au moins un composant de maintien (170, 172) pour le maintien de la partie à sectionner (16) dans la réception (174) d'élément objet de la coupe avant et/ou après sectionnement de l'élément objet de la coupe (18), un dispositif de déformation (180) destiné à déformer la partie (16) de l'élément objet de la coupe (18) maintenue dans la réception (174) d'élément objet de la coupe et à sectionner étant prévu, ledit dispositif de déformation (180) comprenant au moins un élément de déformation (176, 178) déplaçable par rapport à la réception (174) d'élément objet de la coupe lors du passage de la position de repos à la position de coupe, et l'élément ou les éléments de déformation (176, 178) étant déplaçables au moins en partie jusque dans la réception (174) d'élément objet de la coupe définie en position de repos lors du passage de la position de repos à la position de coupe, **caractérisé en ce qu'**au moins une partie (192) de l'élément ou des éléments de déformation (176, 178) est montée de manière rotative autour d'un axe longitudinal d'élément de déformation.

2. Instrument de coupe selon la revendication 1, **caractérisé en ce que** l'élément ou les éléments de déformation (176, 178) délimitent au moins en partie la réception (174) d'élément objet de la coupe.

3. Instrument de coupe selon la revendication 1 ou 2, **caractérisé en ce qu'**un premier élément de déformation (176) est plus espacé d'une extrémité (164) distale de l'instrument de coupe (10) qu'un deuxième élément de déformation (178).

4. Instrument de coupe selon l'une des revendications précédentes, **caractérisé en ce que** l'élément ou les éléments de déformation (176, 178) sont réalisés sous forme d'un axe de maintien (198).

5. Instrument de coupe selon l'une des revendications précédentes, **caractérisé en ce que** l'élément ou les éléments de déformation (176, 178) sont dans leur ensemble rotatifs autour de l'axe longitudinal (186) d'élément de déformation.

6. Instrument de coupe selon l'une des revendications précédentes, **caractérisé en ce que** l'élément ou les éléments de déformation (176, 178) comprennent un composant de palier (182) et un composant de déformation (192), et **en ce que** le composant de déformation (192) sur le composant de palier (182) est monté de manière rotative autour de l'axe longitudinal (186) d'élément de déformation.

7. Instrument de coupe selon la revendication 6, **caractérisé en ce que** le composant de palier (182) est réalisé sous la forme d'un tourillon cylindrique (184), et **en ce que** le composant de déformation (192) est réalisé sous la forme d'un manchon de déformation (194) monté de manière rotative sur le tourillon cylindrique (184).

8. Instrument de coupe selon la revendication 6 ou 7, **caractérisé en ce que** les mâchoires de coupe (22, 24) sont disposées ou montées de manière à pouvoir pivoter l'une par rapport à l'autre autour d'un premier axe de pivotement (20), **en ce que** l'élément ou les éléments de déformation (176, 178) sont montés de manière à pouvoir pivoter autour du premier axe de pivotement (20), **en ce que** deux leviers pivotants (32, 34) montés de manière à pouvoir pivoter autour du premier axe de pivotement (20) sont prévus, sur chacun desquels une mâchoire de coupe (22, 24) est disposée ou réalisée, et **en ce que** l'élément ou les éléments de déformation (176, 178) sont disposés ou réalisés sur les leviers pivotants (34).

9. Instrument de coupe selon l'une des revendications précédentes, **caractérisé par** un dispositif d'amortissement des impulsions de coupe (200) pour l'amortissement d'une impulsion de coupe active sur une partie sectionnée (16) de l'élément objet de la coupe (18) lors du sectionnement, le dispositif d'amortissement des impulsions de coupe (200) comportant au moins un élément d'amortissement des impulsions de coupe (202, 204) monté de manière mobile sur l'instrument de coupe (10) et délimitant au moins en partie la réception (174) d'élément objet de la coupe.

10. Instrument de coupe selon l'une des revendications précédentes, **caractérisé en ce que** chaque tranchant (26, 28) présente une première surface latérale (150) orientée en direction distale ou sensiblement distale, et une deuxième surface latérale (152) orientée en direction proximale ou sensiblement proximale.

11. Instrument de coupe selon la revendication 10, **caractérisé en ce que** la première surface latérale (150) définit un premier plan de tranchant (154), qui en position de coupe croise perpendiculairement ou sensiblement perpendiculairement un axe longitudinal (156) défini par l'instrument de coupe (10).

12. Instrument de coupe selon la revendication 10 ou 11, **caractérisé en ce que** la deuxième surface latérale (152) définit un deuxième plan de tranchant (158a, 158b), qui en position de coupe est incliné par rapport à un axe longitudinal défini par l'instrument de coupe (10).

13. Instrument de coupe selon l'une des revendications 10 à 12, **caractérisé en ce que** les deux deuxièmes surfaces latérales (152) des deux tranchants (26, 28) délimitent en position de coupe un évidement (160) en forme de coin orienté en direction proximale, et **en ce que** ledit évidement (160) définit un angle d'ouverture (162).

14. Instrument de coupe selon l'une des revendications 10 à 13, **caractérisé en ce qu'**une extrémité (164) distale de l'instrument de coupe (10) présente un évidement (166) en forme de rainure en position de coupe, et **en ce que** les premières ou les deuxièmes surfaces de coupe définissent ou délimitent au moins en partie l'évidement.

15. Instrument de coupe selon l'une des revendications précédentes, **caractérisé par** une partie de saisie et d'actionnement (127) formant une extrémité proximale de l'instrument de coupe (10), et en ce que ladite partie de saisie et d'actionnement (127) comprend deux branches d'actionnement (88, 90) montées de manière pivotante l'une par rapport à l'autre autour d'un deuxième axe de pivotement (92) pour le déplacement des mâchoires de coupe (22, 24) de la position de repos vers la position de coupe.
